# EUROPEAN PATENT SPECIFICATION

(11) **EP 2 598 632 B1**
(45) Date of publication and mention of the grant of the patent: **17.09.2014**
(21) Application number: 11754758.8
(22) Date of filing: 27.07.2011
(51) Int. Cl.: C12N 5/073, A61K 35/50, C12N 5/074

(54) **ISOLATION OF PLURIPOTENT STEM CELLS FROM AMNIOTIC FLUID AND USE THEREOF IN THE TREATMENT OR PREVENTION OF AUTOIMMUNE DISEASES**
VERFAHREN ZUR ISOLIERUNG PLURIPOTENTER STAMMZELLEN AUS DEM FRUCHTWASSER UND DEREN VERWENDUNG ZUR BEHANDLUNG ODER VORBEUGUNG VON AUTOIMMUNKRANKHEITEN
PROCEDE D'ISOLATION DE CELLULES SOUCHES PLURIPOTENTES DU LIQUIDE AMNIOTIQUE ET LEUR UTILISATION DANS LE TRAITEMENT OU LA PREVENTION DES MALADIES AUTOIMMUNES

(30) Priority: 27.07.2010 IT RM20100416
(43) Date of publication of application: 05.06.2013
(73) Proprietor: Universita' Degli Studi Dl Perugia, 06123 Perugia (IT)
(72) Inventor: ROMANI, Rita, 06123 Perugia (IT); FALLARINO, Francesca, 06123 Perugia (IT); PUCCETTI, Paolo, 06123 Perugia (IT); PIRISINU, Irene, 06123 Perugia (IT); ROSI, Gabriella, 06123 Perugia (IT); DONTI, Emilio, 06123 Perugia (IT); BISTONI, Giovanni, 06123 Perugia (IT)
(74) Representative: Gitto, Serena
(86) International application number: PCT/IT2011/000270
(87) International publication number: WO 2012/014247

(56) References cited:
- SESSAREGO NADIA ET AL: "Multipotent mesenchymal stromal cells from amniotic fluid: solid perspectives for clinical application.", HAEMATOLOGICA MAR 2008 LNKD- PUBMED:18268281, vol. 93, no. 3, March 2008 (2008-03), pages 339-346, XP002622816, ISSN: 1592-8721
- ROELEN DAVE L ET AL: "Differential immunomodulatory effects of fetal versus maternal multipotent stromal cells.", HUMAN IMMUNOLOGY JAN 2009 LNKD- PUBMED:19010366, vol. 70, no. 1, January 2009 (2009-01), pages 16-23, XP025860526, ISSN: 0198-8859
- TSAI MING-SONG ET AL: "Isolation of human multipotent mesenchymal stem cells from second-trimester amniotic fluid using a novel two-stage culture protocol.", HUMAN REPRODUCTION (OXFORD, ENGLAND) JUN 2004 LNKD- PUBMED:15105397, vol. 19, no. 6, June 2004 (2004-06), pages 1450-1456, XP002404972, ISSN: 0268-1161
- ORCIANI M ET AL: "Potential role of culture mediums for successful isolation and neuronal differentiation of amniotic fluid stem cells.", INTERNATIONAL JOURNAL OF IMMUNOPATHOLOGY AND PHARMACOLOGY 2008 JUL-SEP LNKD- PUBMED:18831926, vol. 21, no. 3, July 2008 (2008-07), pages 595-602, XP009111425, ISSN: 0394-6320

### SEQUENCE LISTING

<110> università degli Studi di Perugia
<120> isolation of pluripotent stem cells from amniotic fluid and use thereof in the treatment or prevention of autoimmune diseases
<130> PCT28368
<150> RM2010A000416
   <151> 2010-07-27
<160> 24
<170> PatentIn version 3.5
<210> 1
   <211> 25
   <212> DNA
   <213> artificial
<220>
   <223> OCT-4 forward primer
<400> 1
   cgtgaagctg gagaaggaga agctg 25
<210> 2
   <211> 25
   <212> DNA
   <213> artificial
<220>
   <223> OCT-4 reverse primer
<400> 2
   caagggccgc agcttacaca tgttc 25
<210> 3
   <211> 25
   <212> DNA
   <213> artificial
<220>
   <223> NANOG forward primer
<400> 3
   cagccccgat tcttccacca gtccc 25
<210> 4
   <211> 25
   <212> DNA
   <213> artificial
<220>
   <223> NANOG reverse primer
<400> 4
   cggaagattc ccagtcgggt tcacc 25
<210> 5
   <211> 26
   <212> DNA
   <213> artificial
<220>
   <223> SOX2 forward primer
<400> 5
   gggaaatggg aggggtgcaa aagagg 26
<210> 6
   <211> 26
   <212> DNA
   <213> artificial
<220>
   <223> SOX2 reverse primer
<400> 6
   ttgcgtgagt gtggatggga ttggtg 26
<210> 7
   <211> 27
   <212> DNA
   <213> artificial
<220>
   <223> FGF5 forward primer
<400> 7
   gctgtgtctc aggggattgt aggaata 27
<210> 8
   <211> 26
   <212> DNA
   <213> artificial
<220>
   <223> FGF5 reverse primer
<400> 8
   tatccaaagc gaaacttgag tctgta 26
<210> 9
   <211> 19
   <212> DNA
   <213> artificial
<220>
   <223> FGF4 forward primer
<400> 9
   ttcttcgggc catgagcag 19
<210> 10
   <211> 20
   <212> DNA
   <213> artificial
<220>
   <223> FGF4 reverse primer
<400> 10
   ccgaagaaag tgcaccaagg 20
<210> 11
   <211> 26
   <212> DNA
   <213> artificial
<220>
   <223> c-MYC forward primer
<400> 11
   gcgtcctggg aagggagatc cggagc 26
<210> 12
   <211> 26
   <212> DNA
   <213> artificial
<220>
   <223> c-MYC reverse primer
<400> 12
   ttgaggggca tcgtcgcggg aggctg 26
<210> 13
   <211> 25
   <212> DNA
   <213> artificial
<220>
   <223> KLF4 forward primer
<400> 13
   acgatcgtgg ccccggaaaa ggacc 25
<210> 14
   <211> 28
   <212> DNA
   <213> artificial
<220>
   <223> KLF4 reverse primer
<400> 14
   tgattgtagt gctttctggt cgggctcc 28
<210> 15
   <211> 25
   <212> DNA
   <213> artificial
<220>
   <223> beta actin forward primer
<400> 15
   tggcaccaca ccttctacaa tgagc 25
<210> 16
   <211> 24
   <212> DNA
   <213> artificial
<220>
   <223> beta actin reverse primer
<400> 16
   gcacagcttc tccttaatgt cacg 24
<210> 17
   <211> 20
   <212> DNA
   <213> artificial
<220>
   <223> ALP forward primer
<400> 17
   gacatcgcct accagctcat 20
<210> 18
   <211> 20
   <212> DNA
   <213> artificial
<220>
   <223> ALP reverse primer
<400> 18
   tcacgttgtt cctgttcagc 20
<210> 19
   <211> 21
   <212> DNA
   <213> artificial
<220>
   <223> Nodal forward primer
<400> 19
   acatcacttg ccagacagaa g 21
<210> 20
   <211> 21
   <212> DNA
   <213> artificial
<220>
   <223> Nodal reverse primer
<400> 20
   ccaacaggat taggacactc g 21
<210> 21
   <211> 22
   <212> DNA
   <213> artificial
<220>
   <223> IDO1 sense primer
<400> 21
   tgtctggctg gaaaggcaac cc 22
<210> 22
   <211> 20
   <212> DNA
   <213> artificial
<220>
   <223> IDO1 reverse primer
<400> 22
   ttcccgcagg ccagcatcac 20
<210> 23
   <211> 20
   <212> DNA
   <213> artificial
<220>
   <223> IDO2 sense primer
<400> 23
   tcacccacca ggccaccaca 20
<210> 24
   <211> 20
   <212> DNA
   <213> artificial
<220>
   <223> IDO2 reverse primer
<400> 24
   ttggcaggac ctctgcaggc 20

## Description

The present invention concerns the isolation of pluripotent stem cells from amniotic liquid, a new type of thus isolated pluripotent stem cells and use thereof in the treatment or prevention of autoimmune diseases, as for example type I diabetes, or inflammatory pathologies.

The immune system is characterized by a delicate equilibrium of tolerance condition against *self* antigens with immunity state towards pathogenic antigens. Induction of immunological tolerance is an important phenomenon in treatment and prevention of autoimmune pathologies, wherein an uncontrolled response of immune system against exogenous and *self* antigens is elicited. In fact, it is known that some *subset* of self-reactive T lymphocytes can escape to thymus deletion by reduction of antigen affinity and migrating at peripheral level. After activation, these T lymphocytes can result in autoimmunity. The function regulation and activation of T lymphocytes, therefore, determines the equilibrium between tolerance and autoimmunity and must be considered when therapeutic strategies aiming to modulate the reactivity of T cells are studied (Puccetti P. et al. 2007 Nat. Rev. Immunol 7:817-823)

Type I diabetes is considered a prototype of human autoimmune diseases. This autoimmune pathology affects β cells of insulin producing pancreatic islands (Katz J.D. et al. 1993 Cell 74:1089-1100). Recent studies have pointed out that the modulation of island directed autoimmune response, by activation of specific *pathway* involved in the control of tolerance, could result not only in the modulation of autoimmune response but also regeneration of pancreatic islands (O'Neill K.E et al. 2008 Bioessays 30:617-620). Therefore, new therapeutic approaches, aiming to modulate the autoimmune response in diabetics are interesting objects, because the same could be associate to restoration of pancreas β cell function in diabetics (Hardikar A.A. 2004 Trends Endocrinol. Metab. 15:198-203; Dor Y., et al. 2007 Sci STKE (414):pe66). It has been suggested that human mesenchymal stem cells (MSC) could be a valid therapeutic tool for activation of this double function. Recent studies have demonstrated the ability of these cells to prevent autoimmune pathologies (Gordon D. et al. 2008 Neurosci. Lett 448:71-73; Gonzalez M.A. et al. 2009 Arthritis Rheum 60:1006-1019). Among the mechanisms the activation of these effector immunomodulatory responses is based on, a predominant role would be carried out by the activation of indoleamine- 2,3-dioxigenase (IDO) enzyme in MSCs and especially in human ones (Jones B.J. et al. 2007 Placenta 28:1174-1181). IDO is an intracellular enzyme containing an heme group catalyzing the starting reaction and limiting the tryptophan degradation through "kynurenine pathway" (Grohmann U. et al. 2003 Trends Immunol. 24:242-248) IDO is expressed at high levels in several human tissues as well as in macrophages and dendritic cells, and it is induced in various inflammatory states by IFN-γ and other pro-inflammatory cytokines, it can be considered as an important effector system for the regulation of innate and adaptive immune response. Although it represents only one of numerous ways to induce tolerance, it has been demonstrated that immunosuppressive pathway of tryptophan catabolism contributes to a successful pregnancy and implants as well as an effective control of autoimmunity (Katz J.D. et al. 1993 Cell 74:1089-1100).

The term "stem cells" refers to cells that are able to proliferate indefinitely and to be differentiated in one or more cell types (Mimeault M. et al. 2006 Stem Cells 24:2319-2345). Stem cells commonly are classified based on their possible development from totipotency to unipotency. A totipotent stem cell, represented in mammals by zygote or premature blastomer, has the potential to generate a complete organism, including all the tissues both embryonic (somatic and stem line) and extra-embryonic. Pluripotent stem cells, including embryonic ones, can be differentiated in all organism cell types, derived from three germinal layers, but the same cannot result in a complete organism. Multipotent stem cells, identified in several tissues of endodermic, mesodermic and ectodermic origin, have the capacity to generate a limited set of cell lines, and unipotent ones can be only developed in mature cell type (Solter D. 2006 Nat Rev Genet. 7:319-327). The stem cells depending on origin thereof can be classified in two groups, embryonic (ESC) or adult stem cells (ASC). ESCs are pluripotent, derive from blastocyst internal cellular mass in early steps of embryonic development (Cole R.J. et al. 1965, in Preimplantation Stages of Pregnancy (Wolstenholme, G. E. W. & O'Connor, M. eds) 82-122 (Churchill, London, 1965); Cole R.J. et al. 1966 Dev. Biol. 13:385-407; Evans M.J. et al. 1981 Nature 292:154-156; Bongso A. et al. 1994 Hum. Reprod. 9:2110-2117; Thomson J.A. et al. 1998 Science 282:1145-1147). ECSs express cell surface antigens (SSEA-3, SSEA4, TRA1-60, TRA1-81, BETWEEN 2-54) and transcription specific factors (OCT-4, NANOG, SOX-2, FGF4, REX1). In addition, ESCs are suitable, *in vitro,* to split symmetrically, indefinitely, without differentiation and are suitable to originate teratocarcinoma in immunodepressed rats. Ethical, moral, political problems and tumorigenicity currently limit the use of these cells for cell therapy.

ASCs reside in specific niches occurring in nearly all the adult tissues and are able to maintain their multipotency (Jones D.L. et al. 2008, Nat Rev Mol Cell Biol. 9:11-21). The differentiation potential and proliferation capacity thereof are limited when compared to embryonic ones.

During the last few years in order to overcome limits concerning the use of both ESCs and ASCs, alternative sources of stem cells, represented by re-programmed adult cells, named induced pluripotent stem cells (iPS), and mesenchymal stem cells, have been searched. iPS cells are "molecularly" and functionally indistinguishable from ESCs, currently numerous studies aiming to estimate the safety of such cells are carried out. MSCs are multipotent stem cells, which can be isolated from several tissues, including bone marrow, fat tissues, placenta and amniotic liquid (Bianco P. et al. 2000 J. Clin. Invest. 105:1663-1668; Im G.I. et al. 2005 Osteoarthritis Cartilage 13:845-53; Anker P.S. et al. 2004 Stem Cells 22:1338-1345). MSCs are well known for the potential to originate a large number of cell lines and in recent years the characterization thereof (Dominici M. et al. 2006 Cytotherapy 8:315-317) allowed the clinic use. The therapeutic applications of MSCs yet are limited due to low number of cells obtainable from various adult tissues, reduced proliferation ability in addition to withdrawal invasivity, particularly medullary aspirates (Rao M.S. et al. 2001 Mech. Ageing Dev. 122:713-734). Other sources of stem cells as identified during the last few years and not controversial are foetal membranes (placenta) and amniotic liquid. This last source of stem cells is particularly interesting since the use of residual material of genetic diagnosis pre-natal route does not involve any ethical, moral or political problem. It is possible to isolate from amniotic liquid multipotent and/or pluripotent stem cells having duplication times comparable to embryonic ones, without specific manipulation need as for iPS cells.

Two types of stem cell population have been isolated from amniotic liquid up to now: i.e. mesenchymal stem cells (AFMSC) and stem cells (AFS). Both can be used as primary cells (not transformed or immortalized) without further technical manipulations. AFMSCs exhibit typical MSC characteristics: morphology similar to fibroblast, clonogenic ability, multipotency, immunosuppressive properties, expression of membrane antigen and typical mesenchymal genes. On the other hand, AFSs are pluripotent cells with ESC and ASC intermediate characteristics (Bajada S. et al. 2008 J Tissue Eng. Regen Med. 2:169-83; Siegel N. et al. 2007 Stem Cell Rev. 3:256-264; Cananzi M. et al. 2009 Reprod. Biomed. Online 18 Suppl 1:17-27). Said cell express both ESC markers and MSC markers and functional characteristics. The same are able to differentiate in cellular lines representing all the embryo and do not form tumours after *in vivo* implant (Cananzi M. et al. 2009 Reprod. Biomed. Online 18 Suppl 1:17-27)

There are various methods for isolation of said two types of stem cells from amniotic liquid (Sessarego N. et al. 2008 Haematologica 93:339-346; Roelen D.L. et al. 2009 Human Immunology 70:16-23; Orciani M. - Di Primio et al. 2008 Int. J. Immunopathol. Pharmcol. 21:595-602). The most commonly used protocol is according to De Coppi (De Coppi P. et al. 2007 Nature Biotechnology 25:100-106; Patent to Atala A. and De Coppi P. "Methods of isolation, expansion and differentiation of fetal stem cells from chorionic villus, amniotic liquid, and placenta and therapeutic uses thereof EP1442115), based on two steps, firstly immunological selection of c-kit positive cells (CD117) and a successive culture expansion of such cells.

Other isolation procedures do not involve initial immunological selection, but disposition of cells in 3-5 ml of fresh amniotic liquid for culture in media specific for growth of mesenchymal stem cells (Orciani M. et al. 2008 Int. J. Immunopathol. Pharmcol. 21:595-602)

An isolation procedure not using fresh amniotic liquid is reported by Tsai (Tsai M.S. et al. 2004 Hum. Reprod. 19:1450-1456; patent entitled "Two-stage culture protocol for isolating mesenchymal stem cells from amniotic liquid" US patent n° 101,710,B2 on sep. 5th, 2006). Tsai procedure involves the use of suspended cells after primary culture resulting from cytogenetic pre-natal diagnosis protocol; said cell successively are centrifuged and cultured in specific medium.

The just described isolation procedures suffer from some disadvantages:
- Subtraction of biological material to pre-natal diagnosis. Amount of withdrawn amniotic liquid according to routine pre-natal diagnosis is approximately 15-20 ml. For isolation of stem cells according to De Coppi and Orciani-Di Primio protocol, an amniotic liquid portion is directly withdrawn or an increased liquid amount must be provided at amniocentesis occurrence. Where a reduced cellular growth occurs, the material subtraction could be penalizing for diagnostic purposes. Procedure according to Tsai uses cells which in pre-natal diagnosis are used in order to have another indispensable source of usable cells for possible further primary genetic analysis investigations.
- The stem cell selection using c-kit according to De Coppi protocol uses a commercially available kit involving a simple but expensive technique (commercial kit is very expensive) compared to author's proposal.
- All the known procedures select stem cells (AFMSC, AFS) before the result of pre-natal diagnosis is known, i.e. the analysis is carried without knowledge whether these cells will display chromosomal or genetic pathologies.

In the light of above it is therefore apparent that there is a need to provide for new isolation procedure of stem cells overcoming the disadvantages of known ones.

The authors of the present invention now have developed a procedure for isolation stem cells from heterogeneous cellular mixture of amniotic liquid. The inventors are able to obtain stem cells from amniotic liquid (HA-SC) independently from starting material. i.e. fresh amniotic liquid or culture residue at the end of diagnostic pre-natal route. Differently than other procedures, being able to obtain HA-SCs at the end of pre-natal diagnosis allows the genetic constitution of amniocytes to be known, before the procedures to obtain HA-SC start, thus avoiding to treat cultures unusable for cellular therapy or storage in stem cell bank. The method proposed by the inventors further is economic both in terms of time and money compared to known procedure. More particularly the inventors are able to isolate two populations of pluripotent stem cells (HA-SC), preponderant phenotypic difference thereof being different proliferation ability. A population presents duplication times of approximately 10-18h while the other of approximately 30-36h under same culture conditions. The two cellular populations therefore have been named fast stem cells (*fHA-SC*) from amniotic liquid and slow stem cells from amniotic liquid (*sHA-SC*). Both HA-SCs present characteristics allowing to be used for cellular therapy: i.e. are pluripotent, not transformed in tumour cells but above all can exert an homeostatic localized and IDO-dependent control on peripheral T cell proliferation and survival, promoting also an antigen- and organ-specific tolerance. Both, are further able to produce discrete amounts of TGF-β1 cytokine (beta 1 transforming growth factor) important for immunoregulatory functions. *sHA-SC* cells present many similarities to previously described AFSs including duplication times, while *fHA-SCs* are dissimilar to AFSs just due to remarkable duplication time difference (under the same experimental conditions). *fHA*-*SCs*, due to high duplication potential, represent a stem cell type different to known ones. High proliferation ability makes *fHA*-*SCs* particularly interesting and useful to be used in cellular therapy, since it is possible to obtain high number of cells in short times. This *fHA-SC* peculiar property is, on the other hand, the limitation intrinsic to all other not embryonic stem cells.

According to the present invention, stem cells are isolated from amniotic liquid samples from women at 16^{th} - 17^{th} week of gestation who voluntarily are subjected to amniocentesis for diagnostic reasons and have signed an informed consent. The isolation procedure of stem cells consists of selection of the cells with particular morphology and colony shape. These colonies can be obtained by culture using an aliquot of fresh amniotic liquid or cell culture remaining at the end of the pre-natal diagnosis, i.e. cell cultures of amniotic liquid used as pre-natal diagnosis stock and disposed after analysis result (or report) delivery.

Where a fresh amniotic liquid aliquot is used (3-5 ml), the samples are centrifuged, cells collected and cultured in suitable media for 6-7 days. Examples of suitable culture media are: (1) CHANG; 2) MSCGM^{™} (LONZA); 3) αMEM +Chang: αMEM supplemented with 18%Chang B, 2% Chang C, 15% bovine foetal serum for stem cells (HYCLONE), 1% penicillin-streptomycin, 1% glutamine; 4) DMEM: DMEM supplemented with 10% bovine foetal serum for stem cells (HYCLONE), 1% penicillin-streptomycin, 1% glutamine; 5) αMEM: 10% αMEM supplemented with bovine foetal serum for stem cells (HYCLONE), 1% penicillin-streptomycin, 1% glutamine).

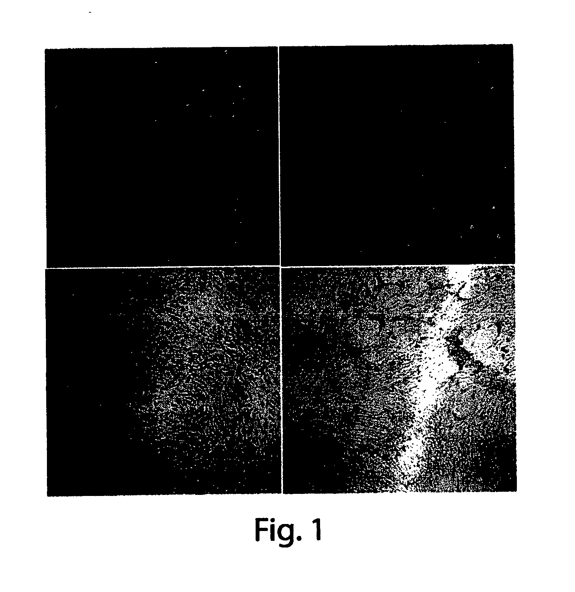

After said culture period only some of cells occurring in amniotic liquid adhere to amniodish® (Euroclone) and originate morphologically different colonies (Figure 1). According to the method of the present invention, only colonies displaying a particular morphology will be selected and cultured in stem cell specific medium.

Cells originating colonies of interest are not exclusive of fresh amniotic liquid but can be found also in residual culture of pre-natal diagnosis. According to pre-natal diagnosis procedure, amniotic liquids (approximately 15-20 ml) are centrifuged, re-suspended in 4 ml of Chang medium, split in 4 primary cultures on amniodish®. After 6 days of culture the cells adhering to amniodish® surface are used for pre-natal analysis, while culture medium, containing some suspended cells from amniotic liquid, are collected and placed in 25 cm² Falcon flask for cell culture, to which 1 ml of Chang fresh medium is added and kept in CO₂ incubator up to diagnosis completion. This culture, named secondary recovery culture, remains for other 6-9 days in CO₂ incubator up to diagnosis completion. During this diagnosis completion period, not previously adhered cells can do and, by replication, originate morphologically different colonies (Figure 1).

As above said, the method according to the present invention can use not only fresh amniotic liquid but also recovery secondary cultures which at the end of preliminary diagnosis are disposed. Below an explicative scheme of pre-natal diagnosis method and starting material used for the isolation of stem cells according to the present invention and known art methods is reported.

| **Starting material usable according to the method of the present invention** | **Pre-natal diagnosis procedure scheme** | **Starting material used according to known art methods** |
|---|---|---|
| X (selection based on colony shape and cell morphology) | Amniotic liquid (15-17 weeks of gestation) | De Coppi: c-kit+ cells immunological selection; Orciani-Di Primio: no selection |
| | ↓ | |
| | Primary culture in amniodish® | Tsai: supernatant use, no selection |
| | ↓ | |
| | supernatant | |
| | Recovery secondary culture in 25 cm² flask | |
| | ↓ | |
| | Completion diagnostic iter | |
| | ↓ | |
| X (selection based on colony shape and cell morphology) | Recovery secondary culture to be disposed at the end of pre-natal diagnosis procedure | |

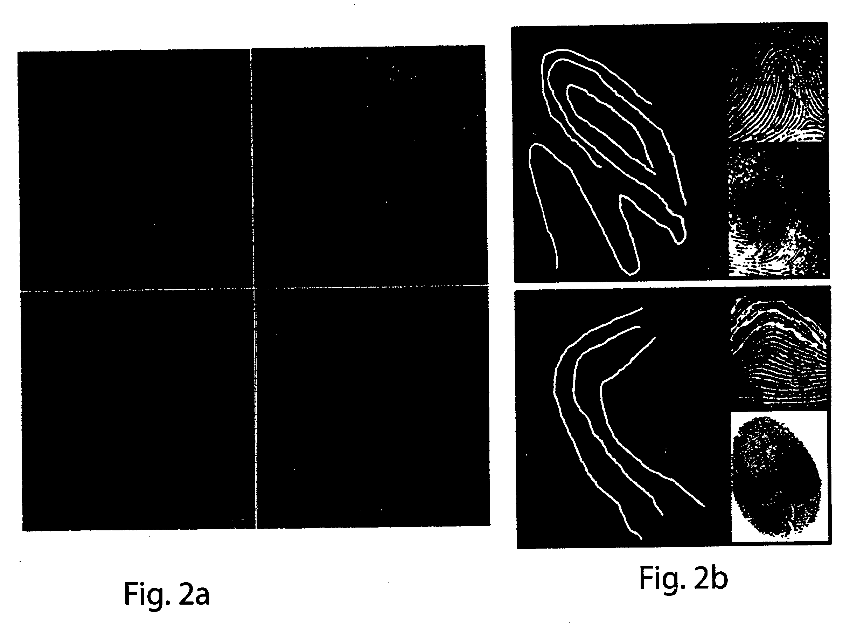

The method according to the invention involves a selection, using inverted optical microscope, based on the inspection of cell morphology, that must be fibroblast-like and colony shape that must be similar as reported in Figure2a, that is resembling arch or tented arch dermatoglyphics. In order for *fHA*-*SCs* to be obtained, only those cultures wherein two colonies having the specific shape as reported in Fig 2a are used, meanwhile all the other cell cultures, not showing the above said colonies, make *sHA-SCs.*

All the cultures cannot be used for *fHA-SC* isolation because not always a sufficient number of colonies is present. The number of cultures from which homogenous *fHA*-*SC* cell lines can be obtained is very low, approximately 1% of amniotic liquids according to author's observation (more than 1000 samples).

In cultures selected from secondary recovery cultures at the end of pre-natal diagnosis procedure Chang culture medium is replaced with stem cell specific medium, as for example, αMEM+Chang, αMEM, DMEM, MSCGM™.

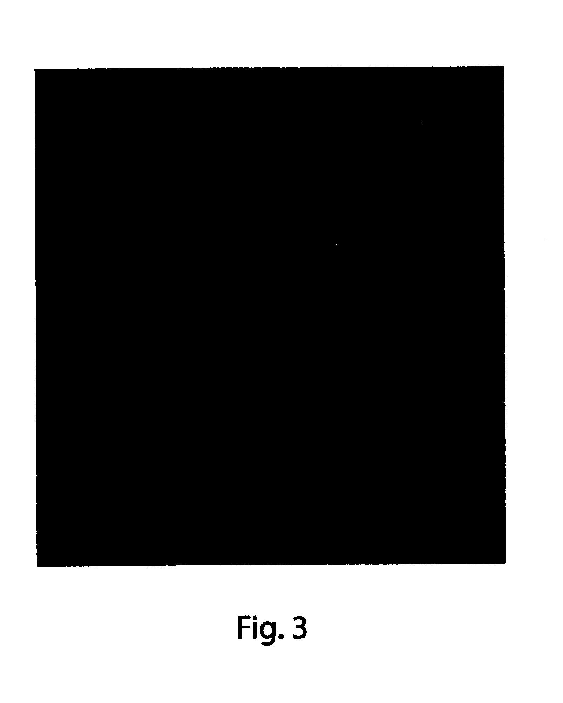

*fHA-SCs* proliferate very quickly, being highly competent with all the other culture cell types, as for example epithelial or *sHA-SC* cells, after at maximum three *in vitro* passages *fHA*-*SCs* represent more than 95% of culture cells (Figure 3) practically becoming a homogenous cell line. *sHA-*SCs, although proliferating in stem cell specific medium, never reach homogeneity. From one amniotic liquid sample it is possible to isolate a single type of independent cell population, not both. The isolation procedure, as above reported, obtains same result using fresh amniotic liquid or residue culture of pre-natal diagnosis route, although the period wherein the material is cultured before the selection is different (6-8 and 18-20 days for first and second cases); this fact does not affect the possibility to isolate HA-SCs, neither the opportunity to obtain from the same sample at various times various HA-SC types.

The procedure of cell isolation according to the present invention offers multiple advantages compared to known techniques, particularly:
1) it is possible for HA-SCs to be selected both from fresh amniotic liquid and at the end of pre-natal diagnosis. The use of secondary recovery culture at the end of pre-natal diagnosis is preferable due to the following reasons:
   a) it does not interfere with normal diagnostic procedures because the selection material is recovered at the end of pre-natal diagnosis in spite of disposal thereof;
   b) it does not require a greater amount of amniotic liquid to be withdrawn by amniocentesis;
   c) the use of cultures at the end of pre-natal diagnosis allows only stem cells from subjects without chromosomal or genetic pathologies to be involved. It is therefore possible to use pathology-free starting material. In addition it results in a further cost reduction, because the isolation of cultures potentially unusable for cellular therapy is avoided. Further, it is also possible pathologies detected during routine genetic surveying to be based on for research experimental model;
   d) it increases the percentage of stem cells occurring in starting sample. The usability of recovery cultures originating from 15-20 ml of amniotic liquid increases the number of stem cells occurring in starting sample, rather than 3-5 ml of fresh amniotic liquid.
2) the process of the invention offers the possibility consistent results to be obtained based on microscope selection of cultures.
3) the pre-selection by microscope observation of cell morphology and colony shape is an economic and quick identification system for cultures having higher probability to originate *fHA-SCs.*
4) the amniotic liquid is easy available as the pre-natal diagnosis by amniocentesis is a widely used procedure;
5) the process of the invention overcomes ethical concerns as to embryo disruption for therapeutic or research use.

Both HA-SC cell types (*sHA-SCs, fHA-SCs*), isolated according to the process of the invention, have been phenotypically characterized: growth plots, duplication times, membrane markers, pluripotency markers, immunological properties.

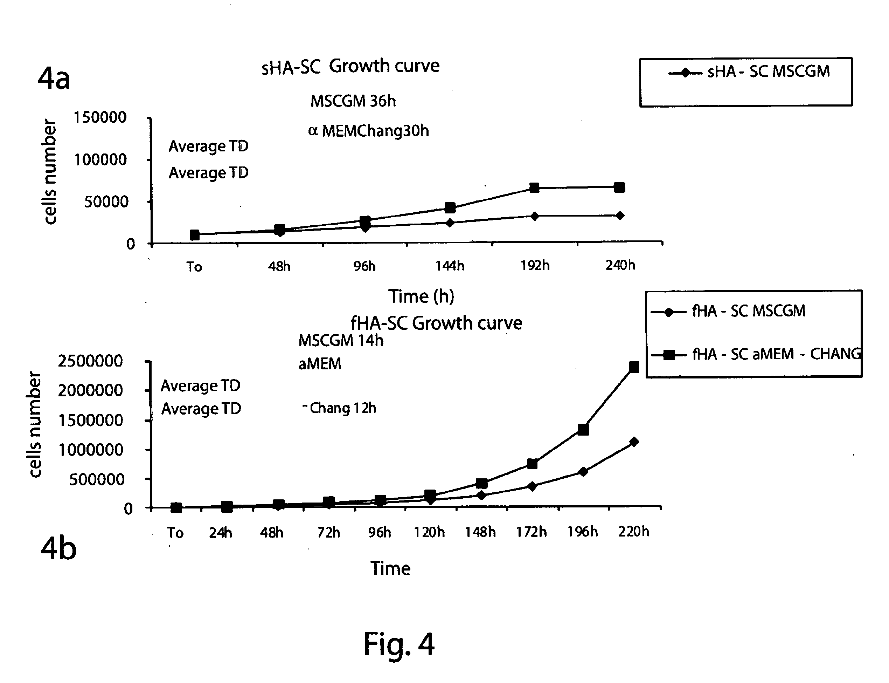

HA-SC duplication ability has been estimated using different culture media and various cell amounts. Figure 4 shows duplication times for said two cell types; the time values are very different for the two cell populations: 30-36h for *sHA-SCs* (Figure 4A), 10-18h for *fHA-SCs* (Figure 4B). As it is possible to see in said Figure cell growth plots using MSCGM and αMEM-Chang media point out the effect of culture medium on duplication ability, but yet the population difference still remains. On the contrary meaningful differences are not detected when different cell amounts are used.

High proliferation ability of *fHA-SC* type cells compared to other not embryonic stem cells, makes the same particularly suitable for any experimental procedure and cell therapy.

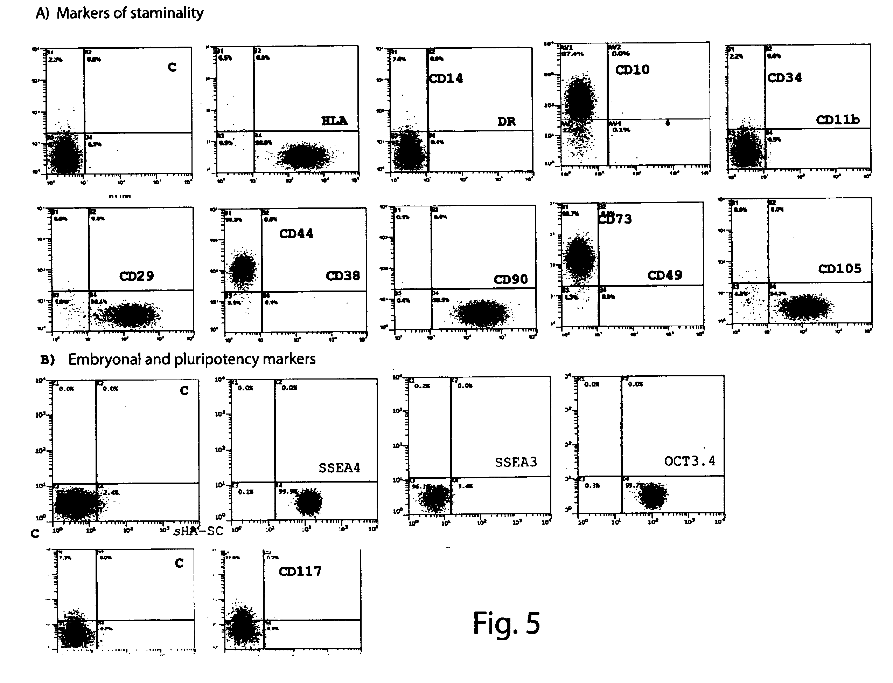

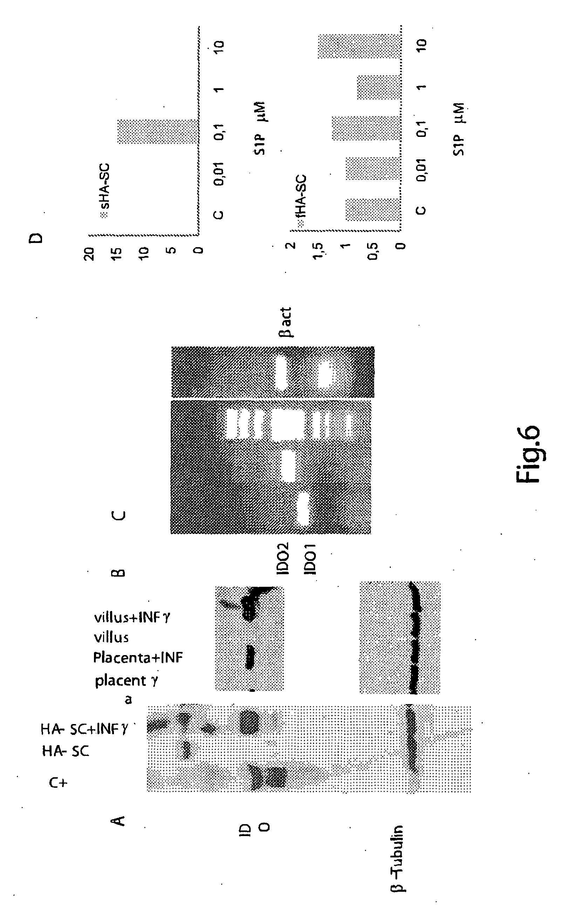

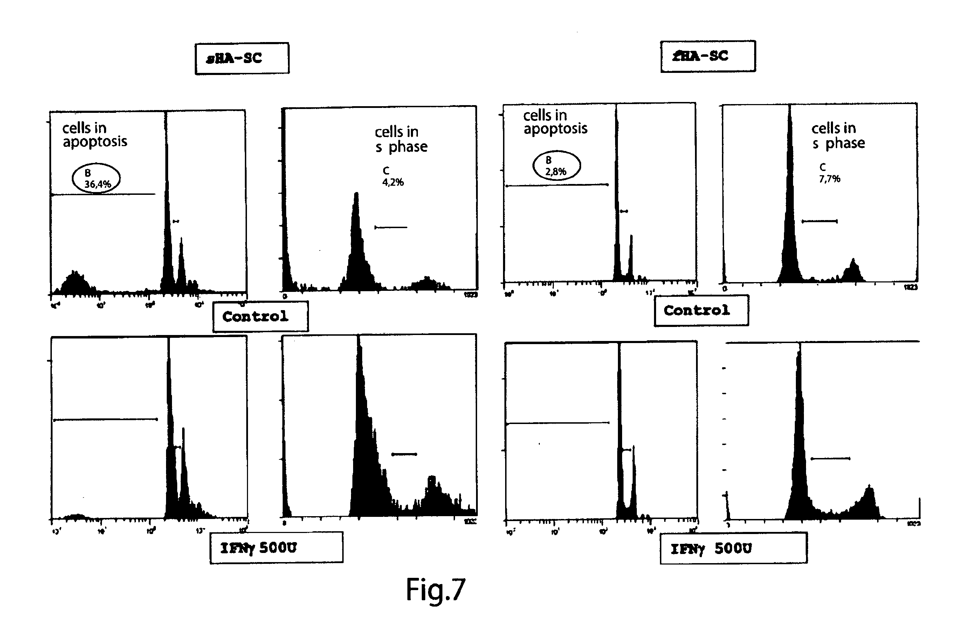

HA-SC cells have the following characteristics:
a) express the following markers: HLA A,B,C; SSEA4; CD10; CD29; CD44; CD73; CD90; CD105; OCT4. On the contrary the same are negatives for: SSEA3; Class II DR; CD 11b; CD14; CD 34; CD38; CD 49d (Figure 5A-B);
b) express messengers for CD117 (c-kit) but it is not always possible to determine corresponding protein; this last one has been detected only in some *sHA-SC* cell lines. CD117 expressing *sHA*-*SC* cell lines did at 22% of cells (Figure5C);
c) are pluripotent cells as express main pluripotency associated transcription factors: i.e. OCT4, NANOG, SOX2, FGF5, FGF4, cMYC, KLF4, alkaline phosphatase (ALP), Nodal. Pluripotency factors are expressed by two populations without meaningful differences, except KLF4 that is expressed at higher levels by *fHA-SCs,* approximately 400-fold than *sHA-SCs*;
d) display intermediate properties compared to embryonic and adult stem cells;
e) do not show any change in cell viability as a result of normal procedures of *in vitro* culture. Anyway said two cell populations differ from each other: *fHA-SCs* can be continuously cultured for over150 generations while *sHA-SCs* do not exceed 20 generations;
f) are not subjected to phenotypic modifications after freezing-thawing cycle also for long periods, i.e. longer than two years;
g) show chromosomal stability following usual *in vitro* culture procedures as well as freezing-thawing cycle;
h) do not show tumorigenicity, as opposed to embryonic stem cells;
i) have a low immunogenic profile, as result of low levels of class I MHC complex antigens and absence of class II MHC complex antigens expression;
l) express high levels of IDO enzyme, particularly following contact with pro-inflammatory stimuli, compared to hematopoietic cell lines as for example dendritic cells and macrophages. Peculiar characteristic of *fHA-SCs* is the constitutive expression of two forms for IDO enzyme (IDO1 and IDO2) (Figure 6). By virtue of IDO presence, HA-SCs assume the role of potent effectors for tolerogenic response in treatment of autoimmune pathologies, as for example diabetes. By localized tryptophan depletion in combination with release of pro-apoptotic metabolites (kynurenine), HA-SCs can exert a renewed homeostatic localized and IDO-dependent control on the proliferation and survival of peripheral T cells, promoting also an antigen- and organ-specific tolerance
m) express high levels of TGFβ1, especially following contact with pro-inflammatory stimuli. The authors demonstrated firstly that HA-SCs can be stimulated by sphingosine-1-phosphate signal molecules to produce IDO and TGFβ1;
n) following stimulation with IFNγ (proapoptotic cytokine) HA-SCs show a percentage reduction of apoptotic cells (Figure7) and, particularly, as to *fHA-SCs* a percentage increase of duplicating cells and KLF4 expression increase is noticed.

In order to be able to identify and classify stem cells according to the invention in an univocal way, multiple phenotypic and functional characteristics of HA-SCs have been compared to AFSs and AFMSCs as described in literature. Particularly the comparison, under the same experimental conditions, has been carried out as to cell morphology, duplication times, surface or intracellular (proteins) antigens, pluripotency molecular markers (mRNA), functional roles in immunoregulation. Comparative experiments are described in example 4 and outlined in table 1. Obtained results suggest that *sHA-SCs* are more similar to already literature described cells while *fHA-SCs* show unique characteristics and represent a new type of stem cells from amniotic liquid.

The comparison of HA-SCs with other stem cell types as literature described shows some differences as to: 1) surface markers, 2) homogeneity of obtained cell cultures, 3) molecular markers, 4) duplication times, 5) immunological properties.

### 1) Surface markers.

Using isolation procedures according to Orciani-Di Primio *et al*. (Orciani et al. 2008 Int. J. Immunopathol. Pharmacol. 21:595-602,) and Tsai et al. (Hum. Reprod. 2004, 19:1450-1456,) CD105 negative AFMSCs are obtained, CD105 is a membrane antigen that, according to guideline of the International Society of Cellular Therapy in MSCs, must be more than 95% positive for subject cells (Dominici M. et al. 2006 Cytotherapy 8:315-317). Moreover, stem cells obtained by Tsai *et al.* are weakly positive for two fundamental antigen, i.e. CD90 and CD117. Low levels of CD44 and CD105 are expressed also by AFMSCs as described by Sessarego et al. (Haematolgica 2008, 93:339-346). HA-SCs do not express CD117 protein, except some *sHA-SC* cultures, contrarily to AFSs obtained according to De Coppi.

### 2) Cell culture homogeneity.

Sassarego et al. (Haematolgica 2008, 93:339-346) asserts that cell lines according to their procedure contain various cell sub-populations different as to dimensions and morphology; analogous result is obtained by the inventors as to *sHA-SCs* while *fHA-SCs* prove to be 95% homogenous (Figure5D).

### 3) Molecular markers.

Tsai et al. (Hum. Reprod. 2004, 19:1450-1456) asserts that AFSMCs isolated according to their procedure express OCT4 mRNA but only in a sub-population it is possible to detect the protein, while all the obtained HA-SCs according to the present invention express always OCT4 protein at a percentage of 99% (Figure5b).

### 4) Duplication times.

Duplication times for HA-SCs, AFSs and AFMSCs under the same experimental conditions have been evaluated; according to the comparison it is possible to deduce that *sHA-SCs* have average duplication times of 30-36h, similar to those already described for AFSs and AFMSCs (Roubelakis M.G. et al. 2007 Stem Cells Dev. 16:931-952; Cananzi M. et al. 2009 Reprod Biomed Online. 18 Suppl 1:17-27), while *fHA-SCs* have duplication times of 10-18h, that are remarkably lower than other cells under any experimental condition.

### 5) Immunological properties.

AFSCs have characteristics comparable to MSCs and therefore often immunosuppressive (Roelen D.L. et al. 2009 Hum. Immunol. 70:16-23), but not immunoregulatory properties have been described. The innovative aspect of HA-SCs according to the present invention consists of immunoregulatory ability in association with differentiation of lymphocyte T specific populations. Roelen et al. (Hum. Immunol. 2009, 70:16-23) have described the ability of AFMSCs to produce IDO but at very low levels, such that it is possible only mRNA and not protein to be detected. On the contrary HA-SCs produce remarkable basal levels of IDO enzyme (IDO1 or IDO2) and after INFγ induction the expression levels reach higher values than hematopoietic cell lines, as for example dendritic or placenta derived cells.

In conclusion, *sHA-SCs* have characteristics similar to cells described in known art, while *fHA-SCs* are a new type of cells. The originality of *fHA-SCs* is due to high duplication ability and the immunosuppressive and immunomodulatory properties thereof.

On the base of these data, a set of studies on NOD mice (non obese diabetic, experimental model type I diabetes) has been carried out in order to verify whether also for a autoimmune origin pathology, i.e. type I diabetes, *fHA-SCs* could exert a therapeutic effect. It is in fact interesting to notice that *fHA-SCs* have auto-protection capacity against rejection although the same represent in immuno-competent mice a xenogenic graft and therefore do not need host immuno-depression. On the other hand, in NOD mice, there are indicia that syngeneic mesenchymal cells (i.e. from mice) exert protection activity.

The results have confirmed this hypothesis, in fact HA-SCs inoculated in NOD mice, after 5 months, have not elicited rejection nor neoplastic growth. HA-SCs have been detected in pancreas (not in lymph node or spleen), where are localized after an *homing* process. In pancreas, target organ of autoimmunity inflammation, these cells induce the reduction of inflammatory processes.

Experimental data obtained for NOD mice demonstrate therefore therapeutic potential of *fHA-SCs,* resulting from the ability to inhibit immunity and inflammatory responses only in target sites and facilitate the damaged tissue repair through migration according to a cytokine gradient within inflammatory areas (*homing* process). *fHA-SCs* according to the present invention therefore are suitable to induce a localized immuno-suppression. Localized immuno-suppression is to be considered much more advantageous for patients than systemic one, because the latter could increase infection risk. Moreover, a local regulation of immunity responses by *fHA-SCs* promotes tissue regeneration, recruiting endogenous stem cells in damaged site and can induce differentiation of local stem cells in adult cells.

In usual cellular therapies there are problems of cell rejection and localization in target organ. These problems are overcome thanks to peculiar characteristics of stem cells according to the invention. In fact, the same having a low immunological profile, are not rejected by the host because are not recognized as foreign (overcoming immune barrier) and thanks to the ability to carry out an *homing* process are able to be localized in target organ independently; these are the fundamental characteristics allowing the use thereof for therapy of autoimmune diseases.

Currently a definitive treatment for autoimmune pathologies is not available, meanwhile an aberrant immune patient response damages, chronically and irreversibly, tissues and vital organs of the subject.

The stem cells from human amniotic liquid have the double scope to reconstitute lost tissue patrimony by supplying a set of specific cell precursors (pluripotent stem cells) and, concurrently, to correct the autoimmunity underlying immunological defect in patients. Data obtained by the authors of the invention suggest that the mere administration of cells obtained according to the particular isolation process results in a physiological re-balance of immune response in diabetic animal.

It is therefore a specific object of the present invention a process for isolation of pluripotent stem cells from an heterogeneous cell mixture of amniotic liquid comprising or consisting of the following steps:
a) taking a secondary recovery culture of cell from amniotic liquid at the end of pre-natal diagnosis procedure or culturing cells from fresh amniotic liquid in suitable culture medium;
b) selecting by optical microscope observation adhered cell cultures having at least two cell colonies displaying a shape similar to arch or tented arch dermatoglyphics;
c) isolating cell cultures selected in step b) and culturing in culture medium for stem cells;
d) carrying out a sufficient passage number in vitro in order at least 90% of stem cells forming cell colonies shaped similarly to arch or tented arch dermatoglyphics to be obtained. Generally 2 or 3 passages are enough in order homogenous cultures to be obtained.

Due to above exposed reasons, for the preparation of human cells, it is preferable to use in step a) a secondary recovery culture of cells from amniotic liquid at the end of pre-natal diagnosis procedure, When pluripotent stem cells are prepared from animals, fresh amniotic liquid can be used.

Culture media suitable for stem cells from amniotic liquid are, for example as below:
- Chang (Chang medium composition Chang medium: salts 7900 mg/l; Dextrose 1400 mg/l; Amino acids 781 mg/l; L-Glutamine 219; Polypeptides 66 mg/l; Vitamins 22 mg/l; Deoxyribonucleosides 21 mg/l; Ribonucleosides 20 mg/l; Sodium Pyruvate 110 mg/l; Others components 8 mg/l; Hormones and trace elements 0,00025 mg/l; Steroidal hormones 0.0013 mg/l; Newborn Calf serum 6% v/v; foetal calf serum 6% v/v. Said medium is commercially available as a kit and consists of two components: freeze-dried supplement (C) and basal medium (B) to be combined;
- MSCGM ^{™} (LONZA);
- αMEM + Chang (αMEM-Chang medium composition: αMEM (Earle's Salts, 25mM HEPES, without L-Glutamine) supplemented with 18% Chang B, 2% Chang C, 15% bovine foetal serum for stem cells (HYCLONE), 1% penicillin-streptomycin, 1% glutamine);
- DMEM (DMEM with 4500 mg/L D-Glucose, sodium pyruvate, without L-Glutamine: DMEM supplemented with 10% bovine foetal serum for stem cells (HYCLONE), 1% penicillin-streptomycin, 1% glutamine);
- αMEM (αMEM medium composition: αMEM supplemented with 10% bovine foetal serum for stem cells (HYCLONE), 1% penicillin-streptomycin, 1% glutamine).

For stem cells MSCGM^{™}(LONZA), αMEM+Chang, DMEM(DMEM, αMEM is preferably used.

Pluripotent stem cells from amniotic liquid obtainable according to the process as above defined represent a further object of the present invention, said cells being characterized in that they form cell colonies having a shape resembling arch or tented arch dermatoglyphics.

The pluripotent stem cells according to the present invention are characterized in that have duplication times of approximately 10±2 hours in alpha-MEM-Chang medium. Moreover, said cells are characterized in that express HLA A,B,C; CD10; CD29; CD44; CD73, CD90; CD105,SSEA4, OCT4, SOX2, NANOG, KLF4, cMyc, FGF4, FGF5, Nodal, ALP markers and do not express Class II DR; CD 11b; CD14; CD 34; CD38; CD49d; CD117; SSEA3 markers.

The present invention further refers to a pharmaceutical composition comprising or consisting of pluripotent stem cells, as above defined, together with one or more pharmaceutically acceptable excipients and/or adjuvants. The pharmaceutical composition can be prepared in the form of implant as, for example, alginate tablets. In addition the composition can further comprise tryptophan metabolites deriving from pluripotent stem cells as above defined.

The pluripotent stem cells or pharmaceutical composition according to the invention can be used in medical and veterinary field, particularly, for the treatment or prevention of autoimmune inflammatory diseases as for example: diabetes, rheumatoid arthritis, multiple sclerosis. In addition the pluripotent stem cells or pharmaceutical composition according to the invention can be used for the prevention against the rejection of organ graft (Graft versus host disease GVHD).

The present invention now will be described by an illustrative, but not limitative, way according to preferred embodiments thereof, with detailed reference to enclosed drawings wherein:
Figure 1. Various types of colonies that can be obtained from amniocyte culture in the procedure of pre-natal diagnosis.
Figure 2. 2a) Cell morphology and shape of colonies to be selected according to the isolation method of pluripotent stem cells according to the invention; 2b) Morphology of colonies to be selected compared to dermatoglyphics: upper panel shows arch, tented arch dermatoglyphics (or mono delta); lower panel shows arch dermatoglyphics (or delta).
Figure 3. Cell culture of fast pluripotent stem cells (*fHA-SCs*) isolated according to the invention after two passages.
Figure 4. Growth plots and duplication times of isolated pluripotent stem cells according to the invention. In figure results obtained using MSCGM and αMEM - Chang media are reported.
Figure 5. Phenotype characterization of pluripotent stem cells according to the invention: 6A) Stem cell markers 6B) Embryonic and pluripotency markers.6C) Expression of CD117 protein in *sHA-SCs.*
Figure 6. Expression of IDO in pluripotent stem cells according to the invention: A) Expression of IDO after INFγ induction B) Expression of IDO in placenta stem cells and corion villi. C) mRNA expression of IDO1 and IDO2 in *fH-SCs.* D) Induction of IDO in HA-SCs after S1 P induction.
Figure 7. Propidium iodide test
Figure 8. Cytofluorimetric assay for the presence of pluripotent stem cells according to the invention (*fHA-SCs*) and lymphocyte infiltrate in the pancreas of NOD mice treated with various *fHA-SC* dose. Pancreas tissues from NOD control mice or treated with 2x10⁶ or 5x10⁶ *fHA-SCs* respectively have been analyzed using CD105 or HLA antibodies specific for human cells (A) the same pancreas tissues have been analyzed for the presence of CD4+ or CD8+ lymphocyte cell infiltrate (B).
Figure 9. *fHA-SCs* transfected with plasmid containing the luciferase gene, inoculated in NOD mice, in caudal region. Cell localization 20 minutes (A) and 30h (B) after the administration is shown. Cells after 30h through homing effect migrated only into the pancreas.

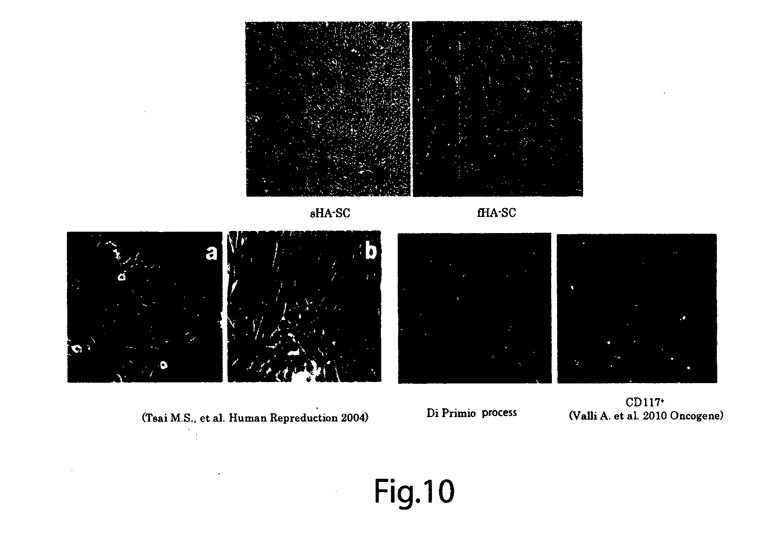

Figure 10. Comparison of various cell morphologies. The comparison of morphologies is carried out using images published by the respective authors except for cells isolated according to Orciani-Di Primio procedure, in this case the inventors have isolated cells according to the procedure and then images have been acquired.

### Example 1: Isolation process of the stem cells from amniotic liquid

The isolation method of stem cells is based on the selection of cells having a colony particular morphology and shape, originating in culture using a portion of fresh amniotic liquid or using cell cultures remaining at the end of pre-natal diagnosis procedure.

### Procedure of pre-natal diagnosis

Amniotic liquids (approximately 15-20 ml) are centrifuged, re-suspended in 4 ml of Chang medium, split in 4 primary cultures on amniodish®. After 6 days of culture the cells adhering to amniodish®, surface are used for pre-natal analysis, while the supernatant, still containing some suspended cells of remaining amniotic liquid, is recovered and placed in 25 cm² cell culture flask, 1 ml of fresh Chang C medium is added and kept in CO₂ incubator until the diagnostic procedures is completed. This culture, named recovery culture, stands for others 6-9 days in CO₂ incubator while diagnosis is completed. During this period, still not adhered cells can adhere and by duplication originate colonies with different morphologies (Figure1).

### Isolation method for pluripotent stem cells according to the invention

From secondary recovery cultures at the end of pre-natal diagnosis procedure, the method according to the invention involves, as a fundamental step, the selection, by observation using optical inverted microscope, of colonies displaying the shape as illustrated in Figure 2a. As shown in Figure 2a colonies to be selected are those having a shape resembling fingerprints (dermatoglyphics). Particularly, the morphology of colonies is similar to arc dermatoglyphics *(or delta) and tented arc (or mono delta)* dermatoglyphics. *Arc or delta* lines go as waves to each other side while *tented arc (or mono delta)* lines are similar to arc but with an increasing stick therebetween (2b). Moreover, the cell morphology of said colonies is similar to fibroblast. In order for *fHA-SCs* to be obtained, only cultures wherein two colonies displaying the above said shape are selected and used; all the other cell cultures, displaying none of above said colonies, originate *sHA*-*SCs*.

Chang culture medium in selected cultures is replaced with a stem cell specific medium (MSCGM, Lonza Company), in any case other stem cell specific media, as for example above described αMEM-Chang, DMEM, αMEM, can be also used.

The cells of above described colonies, in specific medium, already after only three *in vitro* passages amount more than 95% of cells occurring in culture (Figure 3). Selected cultures are further cultured *in vitro* in incubator at 37°C, in 5% CO₂, replacing the medium every three days. Cell freezing is carried out using a mixture containing culture medium supplemented with 50% foetal serum (Hyclone, specific for stem cells), 10% dimethylsulfoxide. First the cells are stored at -80°C for three days and then transferred in the liquid nitrogen containers.

### Example 2: Characterization of cells obtained from isolation process of example 1.

### Determination of duplication time (Figure 4)

Duplication time (TD) has been calculated using the equation TD = *t* × log2/log (*Nt*/*N0*), where *N0* represents the number of seeded cells, *NT is* the number of cells recovered at time t (You Q. et al. 2009. J. Int. Med. Res. 37:105-112). Duplication times have been estimated using following culture media: Chang, DMEM, αMEM-Chang, MSCGM, αMEM, with two different cell amounts: 1000, 2000, 5000 and 10000.

Duplication times obtained using Chang, DMEM, αMEM, αMEM-Chang are very similar while different values have been obtained with MSCGM medium; therefore the successively illustrated data refer to duplication times obtained using αMEM-Chang and MSCGM. *sHA-SCs* have duplication times of 30±3 h in αMEM-Chang and 36±4 h in MSCGM, while *fHA-SCs* have duplication times of 10±2 h in αMEM-Chang and 14±3 h in MSCGM. There are no meaningful differences when different cell amounts are used.

### Phenotype characterization using FACS (Figure 5)

In phenotype characterization using FACS, the cells are labelled with monoclonal antibodies and fluorescence readings are carried out using EPICS cytofluorimeter and EXPO 32 ADC software (Beckman Coulter). 3 x10⁵ cells are labelled with fluorescein isothiocyanate (FITC) or phycoerythrin (PE) conjugated monoclonal antibodies (incubated for 30 minutes at 4°C). Following antibodies were used: HLA A,B,C; class II DR; CD10; CD11b; CD14; CD29; CD34; CD38; CD44; CD 49d; CD73; CD90; CD105;CD117; OCT4; SSEA3; SSEA4. OCT4 is a nuclear protein, therefore Triton X100 0.2% was used for cell permeabilization.

Results obtained using FACS (Figure 5) show that isolated cells are positive for mesenchymal stem cell markers and some embryonic markers, while are negative for main markers of hematopoietic origin stem cells. Particularly, cells are positive for HLA A,B,C; CD10; CD29; CD44; CD73, CD90; CD105,SSEA4, OCT4 while are negative for: Class II DR; CD 11b; CD14; CD 34; CD38; CD49d; CD117; SSEA3.

The determination of some molecular markers, specific for pluripotency being expressed in undifferentiated embryonic cells, is carried out by retro-transcriptase reactions, followed by PCR (RT-PCR). From about approximately 1 x 10⁶ cells, at the same number of passages used for cytofluorimeter analysis, total RNA is extracted using TRIZOL (Invitrogen) and successively RT-PCR is carried out, according to Applied Biosystem protocol using primers for OCT4, NANOG, SOX2, FGF5, FGF4, cMYC, KLF4, alkaline Phosphatase (ALP), Nodal and β-actin positive control. Table 1 shows primer sequences used for RT-PCR.

**Table 1**

| **Primers** | **Sequence** |
|---|---|
| OCT-4 forward | 5' CGTGAAGCTGGAGAAGGAGAAGCTG 3' (SEQ ID NO:1) |
| OCT-4 reverse | 5' CAAGGGCCGCAGCTTACACATGTTC 3'(SEQ ID NO:2) |
| | |
| NANOG forward | 5' CAGCCCCGATTCTTCCACCAGTCCC 3'(SEQ ID NO:3) |
| NANOG reverse | 5' CGGAAGATTCCCAGTCGGGTTCACC 3'(SEQ ID NO:4) |
| | |
| SOX2 forward | 5' GGGAAATGGGAGGGGTGCAAAAGAGG 3'(SEQ ID NO:5) |
| SOX2 reverse | 5' TTGCGTGAGTGTGGATGGGATTGGTG 3'(SEQ ID NO:6) |
| | |
| FGF5 forward | 5' GCTGTGTCTCAGGGGATTGTAGGAATA 3'(SEQ ID NO:7) |
| FGF5 reverse | 5' TATCCAAAGCGAAACTTGAGTCTGTA 3'(SEQ ID NO:8) |
| | |
| FGF4 forward | 5' TTCTTCGGGCCATGAGCAG 3'(SEQ ID NO:9) |
| FGF4 reverse | 5' CCGAAGAAAGTGCACCAAGG 3'(SEQ ID NO:10) |
| | |
| c-MYC forward | 5'GCGTCCTGGGAAGGGAGATCCGGAGC3'(SEQ ID NO:11) |
| c-MYC reverse | 5'TTGAGGGGCATCGTCGCGGGAGGCTG3'(SEQ ID NO:12) |
| | |
| KLF4 forward | 5' ACGATCGTGGCCCCGGAAAAGGACC 3'(SEQ ID NO:13) |
| KLF4 reverse | 5' TGATTGTAGTGCTTTCTGGTCGGGCTCC 3'(SEQ ID NO:14) |
| ALP forward | 5'GACATCGCCTACCAGCTCAT3'(SEQ ID NO:17) |
| ALP reverse | 5'TCACGTTGTTCCTGTTCAGC3 (SEQ ID NO:18) |
| | |
| Nodal forward | 5'ACATCACTTGCCAGACAGAAG3' (SEQ ID NO:19) |
| Nodal reverse | 5'CCAACAGGATTAGGACACTCG3' (SEQ ID NO:20) |
| | |
| β Actina forward | 5' TGGCACCACACCTTCTACAATGAGC 3'(SEQ ID NO:15) |
| β Actina reverse | 5' GCACAGCTTCTCCTTAATGTCACG 3'(SEQ ID NO:16) |

All the cell lines we isolate express pluripotency markers as shown by results in table 2 below.

**Table 2**

| ***Markers*** | ***fHA-Sc*** | ***sHA-SC*** |
|---|---|---|
| OCT-4 | + | + |
| NANOG | + | + |
| SOX2 | + | + |
| FGF4 | + | + |
| FGF5 | + | + |
| c-MYC | + | + |
| KLF4 | ++* | + |
| ALP | + | + |
| Nodal | + | + |

| | | |
|---|---|---|
| **KLF4 expressed in fHA-SCs is 400-fold higher than sHA-SCs.* | | |

### Real-Time RT-PCR

RT-PCR analysis are carried out using Mx3000P instrument (Stratagene, La Jolla, California, USA); the reaction is performed with SYBR Green QPCR Master Mix 2x (Stratagene) and according to suggested protocol. Every sample is standardized based on housekeeping gene thereof (β-actin).

Relative quantification of mRNA from OCT4, NANOG, SOX2, KLF4 genes, is carried out for *sHA-SCs* and *fHA-SCs.* Pluripotency genes are equally expressed in cell lines, with the exception of KLF4, which is expressed mainly by *fHA-SCs,* at level 400 times higher than *sHA-SCs.*

### IDO determination

HA-SCs (1x 10⁶ cells) are pre-treated with INFγ 10 µM or S1P 1 µM overnight. IDO determination is carried out on mRNA using quantitative PCR (Figure 6d) and immunoblotting using human monoclonal antibody (Millipore). Results are standardized by determination of β-Tubulin (Sigma-Aldrich) expression. INFγ or S1 P induced IDO functional capacity is estimated, *in vitro*, dosing the concentration of I-kynurenine, produced by the enzyme as a result of tryptophan hydrolysis. The concentration of I-kynurenine metabolite is determined by HPLC, according to the protocol described by Fallarino *et al.* (Fallarino, F. et al. 2003 Nat. Immunol. 4:1206-1212).

The determination of IDO1 and IDO2 is estimated by RT-PCR using the following primers:
5' TGTCTGGCTGGAAAGGCAACCC 3' IDO1 sense(SEQ ID NO:21)
5' TTCCCGCAGGCCAGCATCAC 3' IDO 1 rev(SEQ ID NO:22)
5' TCACCCACCAGGCCACCACA 3' IDO 2 sense(SEQ ID NO:23)
5' TTGGCAGGACCTCTGCAGGC 3' IDO 2 rev(SEQ ID NO:24)

Figure 6a shows the result of IDO expression in HA-SCs compared to IDO positive control and β- Tubulin. Figure 6b shows the result of IDO expression in placenta and villi stem cells; Figure 6c shows the RT-PCR result with reference to IDO1 and IDO2, Figure 6d shows S1 P IDO induction after β-action normalization.

Average amount of I-kynurenine produced from 1x10⁶ cells in six hours is 0,2± 0,00169 µM.

The production of TGFβ1 is estimated using a specific ELISA kit (Abnova Corporation).

HA-SCs stimulated over 24 h with INFγ show an increase of cellular proliferation; using propidium iodide test, allowing percentages of apoptotic or various step cellular cycle subjected cells to be estimated, it is pointed out that: i) for *sHA*-*SCs* percentage of apoptotic cells decreased from 36% to 10%; ii) for *fHA-SCs* basal percentage of apoptotic cell is very low, i.e. 3%, and after stimulation with INFγ 5% more of cells is divided compared to controls Figure 7. sHA-SCs stimulated over 24h with S1P increase the IDO expression 15 times more than not treated cells, while fHA-SCs show a more limited mRNA increase.

### Example 3: Study on the effects of stem cells inoculation in NOD mice

### NOD Mice

Autoimmune diabetes occurs in 80% of NOD/Mrk female mice at 24^{th} week age. Glycaemia values <200 mg/dl (without glycosuria) and equal to 350-400 mg/dl (stably for at least 1 week) in the presence of glycosuria define pre-diabetic (5-6) and diabetic (>15 weeks) condition. NOD mice maintained over 10 days with blood glucose levels at 350-400 mg/dl change body weight in meaningful way, during various experiments never are treated with exogenous insulin. NOD mice are treated with 2x10⁶ or 5x10⁶ *fHA*-*SCs*, respectively, by percutaneous inoculation. Blood glucose concentrations have been monitored in mice treated once a week as above reported. The expression of typical markers both by HA-SCs and pancreatic lymphocyte infiltrate are analyzed using cytofluorimetry as described in Example 2.

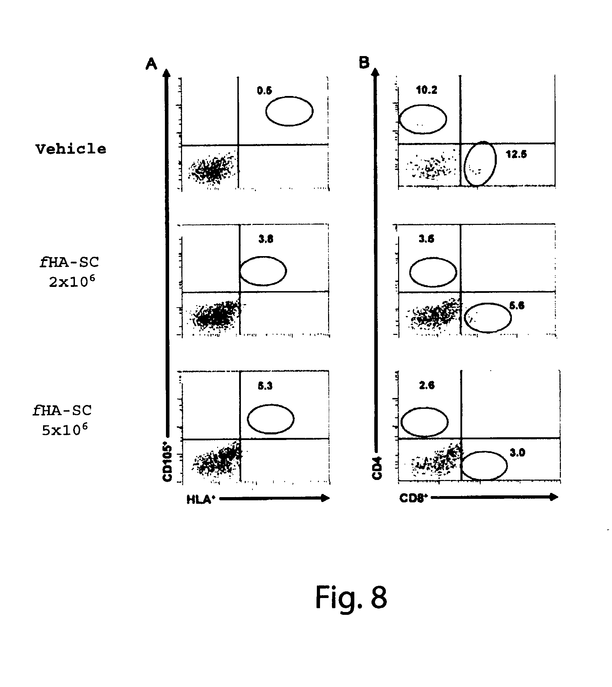

Particularly pancreas samples are withdrawn from pre-diabetic 8-9 week old NOD female mice, displaying at pancreatic level a specific and well characterized leukocyte cell infiltrate, as shown in groups treated only with carrier (fig 8), interestingly mice treated with HA-SCs show meaningfully reduced infiltrates of both CD4+ and CD8+ T cells. In a similar interesting way also after some days it is possible to evidence, by staining with human not murine stem cell specific antibodies, the presence of cells at level of organ wherein inflammation is originated. Such data is of remarkable importance considering that inoculated cells could, on one side, contribute to beta cell regeneration and on the other one, through anti-inflammatory pathway expression, as IDO and cytokine like TGF-β1, regulate at same site the immune responses also in antigen specific way.

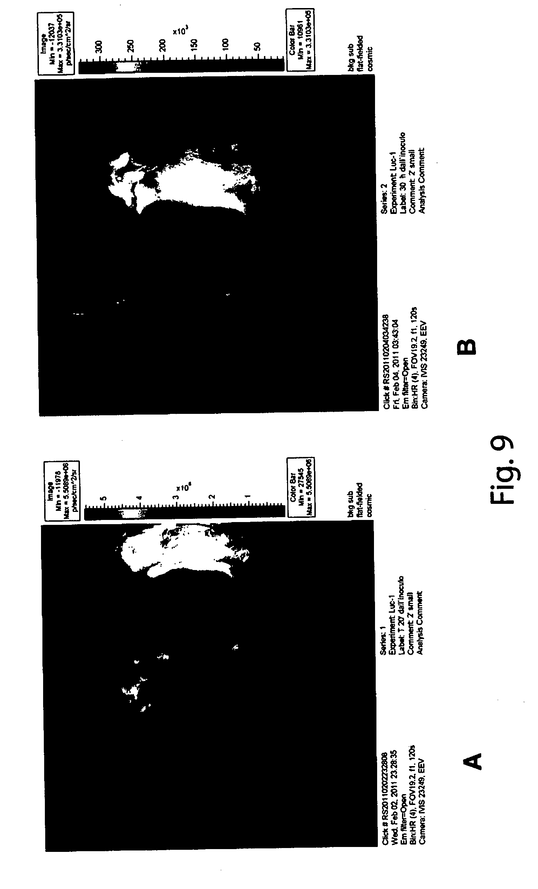

In order to demonstrate HA-SC *homing* process, cells to be inoculated (electroporation) are transfected using a plasmid containing luciferase gene (pGL4.51 (*luc2*/*CMV*/*Neo*) vector). Thus transfected cells after 24h are inoculated in mice caudal region, monitored by animal direct administration of a suitable substrate (vivoglow, Promega) degradable only by luciferase positive cells emitting fluorescent signal successively detected by xenogen Fig9.

### Example 4: Comparative experiments

Phenotype characterization data obtained by the authors, as above described, are compared to literature data relating to AFSs and AFMSCs, by subjecting sHA-SCs and fHA-SCs under experimental conditions as reported in the following papers:
- De Coppi *et al*.;
- Kim et al.(Cell Prolif 2007; 40:75-90.)
- Sessarego *et al*.;
- Orciani *et al.*
- Tsai *et al*.

Results relating to marker expression are reported in Table 3 and 4.

Table 3 compares the expression results of some markers as evaluated by cytofluorimetry.
H: High, L: Low, ND: not detected. The percentage value indicates the percentage of cells expressing the markers in population.

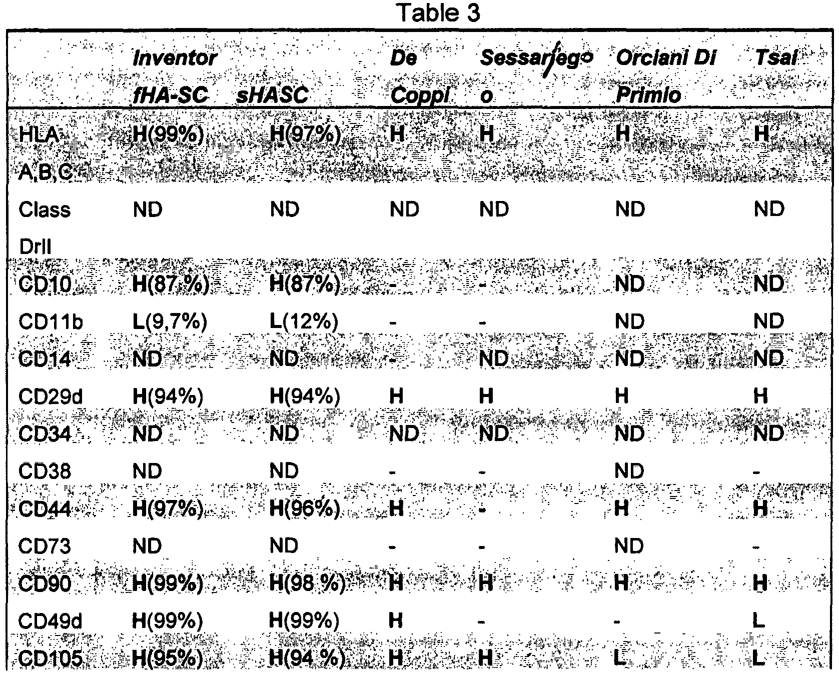

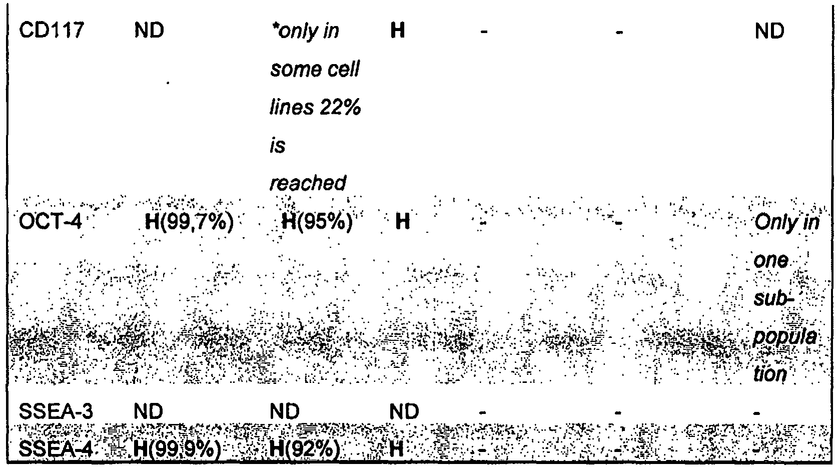

Table 4 compares the expression results of pluripotency molecular markers.

**Table 4**

| | ***Inventors*** | | ***De Coppi*** | ***Kim*** | ***Sessarego*** | ***Orciani Di Primio*** | ***Tsai*** |
|---|---|---|---|---|---|---|---|
| | ***fHA-SC*** | ***sHASC*** | | | | | |
| **Oct-4** | + | + | + | + | NA | NA | + |
| **Sox2** | + | + | NA | NA | NA | NA | NA |
| **Nanog** | + | + | NA | NA | NA | NA | NA |
| **Kit** | ++ | + | NA | NA | NA | NA | NA |
| | | | | | | | |
| **Fgf-4** | + | + | NA | NA | NA | NA | NA |
| **FGF-5** | + | + | NA | + | NA | NA | NA |
| **c-Myc** | + | + | NA | NA | NA | NA | NA |
| **Nodal** | + | + | + | NA | NA | NA | NA |
| **ALP** | + | + | NA | NA | NA | NA | NA |

### 1) Surface Markers.

Using isolation procedures according to Di Primio and Tsai CD105 negative AFMSCs are obtained; moreover, such cells are CD90 and CD117 weakly positive. Low levels of CD44 and CD105 are expressed also from AFMSCs described by Sassarego et al.(Haematolgica 93:339-346, 2008). HA-SCs do not express CD117 protein, except some cultures of *sHA-SCs*, contrarily to AFS isolated by De Coppi.

### 2) Cell culture homogeneity.

Sassarego et al.(Haematolgica 93:339-346, 2008) assert that cell lines as isolated in their studies contain cell sub-populations different as to size and morphology; analogous results are obtained by the inventors as to *sHA-SCs* while *fHA-SCs* display to be 95% homogenous.

### 3) Molecular markers.

Tsai *et al.* (Hum. Reprod. 19:1450-1456,2004) assert that AFSMCs as isolated in their studies express OCT4 mRNA but they are able to detect the protein only in one sub-population, while the inventors are able to detect OCT4 protein in all HA-SCs at 99% percentage (Figure5b).

### 4) Duplication times.

Duplication times for HA-SCs and AFSs and AFMSCs under the same experimental conditions have evaluated, according to the comparison it is argued that *sHA-SCs* have average duplication times of 30-36h, similar to those already described for AFSs and AFMSCs, while *fHA-SCs* have duplication times of 10-18h, i.e. remarkably lower than others, under all the experimental conditions. Duplication times obtained using Chang, DMEM, αMEM (used by Tsai), αMEM-Chang (used by De Coppi and many other authors) media are very similar, while different results are obtained with MSCGM medium (used by Orciani-Di Primio). Successively illustrated data refer to duplication times obtained using αMEM-Chang and MSCGM. *sHA-SCs* have duplication times of 30±3 h in αMEM-Chang and 36±4 h in MSCGM, while *fHA-SCs* have duplication times of 10±2 h in αMEM-Chang and 14±3 h in MSCGM. Some authors assert that duplication times are variable depending on cell concentration (Sassarego N. et al. 2009 Haematologica 93:339-346). The authors estimate growth times using two different cell concentrations, without meaningful differences.

### 5) Immunological properties.

The innovative aspect of HA-SCs consists of immunoregulatory ability in association with differentiation of lymphocyte specific populations. T. Roelen et al. (Hum. Immunol. 2009, 70:16-23) have described the ability of AFMSCs to produce IDO but at very low levels, such that it is possible only mRNA and not protein to be detected. On the contrary HA-SCs produce remarkable basal levels of IDO enzyme (IDO1 or IDO2) and after INFγ induction the expression levels reach higher values than hematopoietic cell lines, as for example dendritic or placenta derived cells.

## Claims

1. Process for isolation of pluripotent stem cells from a heterogeneous cell mixture of amniotic liquid comprising or consisting of the following steps:
a) taking a secondary recovery culture of cells of amniotic liquid at the end of pre-natal diagnosis procedure or culturing cells from fresh amniotic liquid in suitable culture medium;
b) selecting by optical microscope observation adhered cell cultures having at least two cell colonies displaying a shape similar to arch or tented arch dermatoglyphics;
c) isolating cell cultures selected in step b) and culturing them in culture medium for stem cells;
d) carrying out a sufficient passage number in vitro in order to obtain at least 90% of stem cells forming cell colonies shaped similarly to arch or tented arch dermatoglyphics,
wherein said secondary recovery culture of cells of amniotic liquid at the end of pre-natal diagnosis procedure of step a) is obtained from the supernatant of the primary cell culture, said supernatant being separated from said primary cell culture, added with fresh medium and maintained until the end of the pre-natal diagnosis.

2. Pluripotent stem cells of amniotic liquid obtainable according to process as defined in claim 1, said cells being **characterized in that**:
- they form cell colonies displaying a shape resembling that of arch, tented arch. Dermatoglyphics;
- they have duplication times of approximately 10±2 hours in αMEM-Chang medium independently from the number of cultured cells;
- they express HLA A,B,C; CD10; CD29; CD44; CD73, CD90; CD105,SSEA4, OCT4 SOX2, NANOG, KLF4, cMyc, FGF4, FGF5, Nodal, ALP markers and do not express Class II DR; CD 11b; CD14; CD 34; CD38; CD49d;SSEA3 markers;
- they express messengers for CD117 (c-kit);
- they express both IDO1 and IDO2 enzyme;
- they express higher level of IDO enzyme in comparison to hematopoietic cell lines after pro-inflammatory stimuli.

3. Pluripotent stem cells according to claim 2, said pluripotent stem cells being further **characterized in that**:
- they do not show any change in cell viability as a result of *in vitro* culture procedures;
- they can be continuously cultured for over 150 generations;
- they are not subjected to phenotypic modifications after freezing-thawing cycle;
- they show chromosomal stability following *in vitro* culture procedures as well as freezing-thawing cycle;
- they do not show tumorigenicity;
- they can be stimulated by sphingosine-1-phosphate signal molecules to produce IDO and TGFβ1;
- following stimulation with IFNγ, they show a percentage reduction of apoptotic cells, a percentage increase of duplicating cells and an increase of KLF4 expression.

4. Pharmaceutical composition comprising or consisting of pluripotent stem cells as defined in anyone of claims 2-3 together with one or more pharmaceutically acceptable excipients and/or adjuvants.

5. Pharmaceutical composition according to claim 4 in form of an implant.

6. Pharmaceutical composition according to anyone of claims 4-5, further comprising tryptophan metabolites deriving from the pluripotent stem cells as defined in anyone of claims 2-3.

7. Pluripotent stem cells or pharmaceutical composition as defined in claims 2-3 and 4-6, respectively, for use in medical and veterinary field.

8. Pluripotent stem cells or pharmaceutical composition as defined in claims 2-3 and 4-6 respectively, for use in the treatment or prevention of autoimmune diseases.

9. Pluripotent stem cells or pharmaceutical composition for use according to claim 8, wherein the inflammatory autoimmune diseases are selected from the group consisting of diabetes, rheumatoid arthritis, multiple sclerosis.

10. Pluripotent stem cells or pharmaceutical composition as defined in claims 2-3 and 4-6, respectively, for use in the prevention of rejection of organ graft.

## Patentansprüche

1. Verfahren zur Isolierung von pluripotenten Stammzellen aus einer heterogenen Zellmischung aus Fruchtwasser, welches die folgenden Schritten umfasst oder daraus besteht:
a) Bereitstellen einer Sekundärgewinnungskultur von Zellen aus Fruchtwasser am Ende eines pränatalen Diagnoseverfahrens oder Kultivieren von Zellen aus frischem Fruchtwasser in einem geeigneten Kulturmedium;
b) Auswählen, mittels Beobachtung durch ein optisches Mikroskop, von adhärenten Zellkulturen mit mindestens zwei Zellkolonien, welche eine ähnliche Gestalt wie bogenförmige oder gewölbt bogenförmige Dermatoglyphen aufweisen;
c) Isolieren von in Schritt b) ausgewählten Zellkulturen und deren Kultivieren im Kulturmedium für Stammzellen;
d) Durchführen einer ausreichenden Anzahl von Passagen *in vitro,* um mindestens 90 % an Stammzellen, welche Zellkolonien bilden, die ähnlich wie bogenförmige oder gewölbt bogenförmige Dermatoglyphen geformt sind, zu erhalten,
wobei die Sekundärgewinnungskultur von Zellen aus Fruchtwasser am Ende des pränatalen Diagnoseverfahrens von Schritt a) aus dem Überstand der primären Zellkultur erhalten wird, welcher Überstand von der primären Zellkultur separiert wird, einen Zusatz an frischem Medium erhält und bis zum Ende der pränatalen Diagnose aufbewahrt wird.

2. Pluripotente Stammzellen aus Fruchtwasser, erhältlich gemäß dem in Anspruch 1 definierten Verfahren, wobei die Zellen **dadurch gekennzeichnet sind, dass**
- sie Zellkolonien bilden, welche eine Gestalt zeigen, die der von bogenförmigen oder gewölbt bogenförmigen Dermatoglyphen ähnelt;
- sie Verdopplungszeiten von etwa 10 ± 2 Stunden in αMEM-Chang-Medium, unabhängig von der Anzahl der kultivierten Zellen, aufweisen;
- sie die Marker HLA A,B,C; CD10; CD29; CD44; CD73, CD90; CD105, SSEA4, OCT4 SOX2, NANOG, KLF4, cMyc, FGF4, FGF5, Nodal, ALP exprimieren und nicht die Marker Klasse II DR; CD 11b; CD14; CD34; CD38; CD49d; SSEA3 exprimieren;
- sie Messenger für CD117 (c-kit) exprimieren;
- sie sowohl IDO1- als auch IDO2-Enzym exprimieren;
- sie höhere Niveaus an IDO-Enzym im Vergleich zu hämatopoetischen Zelllinien nach entzündungsfördernden Stimuli exprimieren.

3. Pluripotente Stammzellen nach Anspruch 2, wobei die pluripotenten Stammzellen ferner **dadurch gekennzeichnet sind, dass**
- sie keinerlei Änderung der Zelllebensfähigkeit als Folge von *in vitro*-Kulturverfahren zeigen;
- sie kontinuierlich für über 150 Generationen kultiviert werden können;
- sie keinen phänotypischen Modifikationen nach einem Gefrier-Auftau-Zyklus unterworfen sind;
- sie chromosomale Stabilität sowohl nach *in vitro-*Kulturverfahren als auch einem Gefrier-Auftau-Zyklus zeigen;
- sie keine Tumorgenizität zeigen;
- sie durch Sphingosin-1-Phosphat-Signalmoleküle zur Produktion von IDO und TGFβ1 stimuliert werden können;
- sie nach Stimulation mit IFNγ eine prozentuale Verringerung an apoptotischen Zellen, eine prozentuale Erhöhung von duplizierenden Zellen und eine Erhöhung der KLF4-Expression zeigen.

4. Pharmazeutische Zusammensetzung, welche pluripotente Stammzellen wie in irgendeinem der Ansprüche 2-3 definiert zusammen mit einem oder mehreren pharmazeutisch annehmbaren Exzipienten und/oder Adjuvans/Adjuvantien umfasst oder daraus besteht.

5. Pharmazeutische Zusammensetzung nach Anspruch 4 in Form eines Implantats.

6. Pharmazeutische Zusammensetzung nach irgendeinem der Ansprüche 4-5, ferner umfassend Tryptophan-Metabolite, die von den pluripotenten Stammzellen wie in irgendeinem der Ansprüche 2-3 definiert stammen.

7. Pluripotente Stammzellen oder pharmazeutische Zusammensetzung wie in den Ansprüchen 2-3 bzw. 4-6 definiert zur Verwendung auf dem Gebiet der Medizin und Veterinärmedizin.

8. Pluripotente Stammzellen oder pharmazeutische Zusammensetzung wie in den Ansprüchen 2-3 bzw. 4-6 definiert zur Verwendung bei der Behandlung oder Vorbeugung von Autoimmunerkrankungen.

9. Pluripotente Stammzellen oder pharmazeutische Zusammensetzung zur Verwendung nach Anspruch 8, wobei die entzündlichen Autoimmunerkrankungen aus der Gruppe, die aus Diabetes, rheumatoider Arthritis, Multipler Sklerose besteht, ausgewählt sind.

10. Pluripotente Stammzellen oder pharmazeutische Zusammensetzung wie in den Ansprüchen 2-3 bzw. 4-6 definiert zur Verwendung bei der Verhütung einer Organtransplantat-Abstoßung.

## Revendications

1. Procédé pour l'isolement de cellules souches pluripotentes issues d'un mélange de cellules hétérogènes de liquide amniotique comprenant ou constitué par les étapes suivantes :
a) prélever une culture de récupération secondaire de cellules de liquide amniotique à la fin d'une procédure de diagnostic prénatal ou mettre en culture des cellules de liquide amniotique frais dans un milieu de culture adéquat ;
b) sélectionner par observation au microscope optique des cultures de cellules ayant adhéré ayant au moins deux colonies de cellules présentant une forme similaire à des dermatoglyphes en forme d'arc ou d'arc en tente ;
c) isoler les cultures de cellules sélectionnées à l'étape b) et les mettre en culture dans un milieu de culture pour cellules souches ;
d) réaliser un nombre de passages suffisant in vitro afin d'obtenir au moins 90 % de cellules souches qui forment des colonies de cellules ayant une forme similaire aux dermatoglyphes en forme d'arc ou d'arc en tente ;
dans lequel ladite culture de récupération secondaire de cellules de liquide amniotique à la fin de la procédure de diagnostic prénatal de l'étape a) est obtenue à partir du surnageant de la culture de cellules primaire, ledit surnageant étant séparé de ladite culture de cellules primaire, complété avec du milieu frais et conservé jusqu'à la fin du diagnostic prénatal.

2. Cellules souches pluripotentes de liquide amniotique pouvant être obtenues au moyen du procédé selon la revendication 1, lesdites cellules étant **caractérisées en ce que** :
- elles forment des colonies de cellules présentant une forme ressemblant à celle de dermatoglyphes en forme d'arc ou d'arc en tente ;
- elles ont des temps de duplication d'environ 10 ± 2 heures dans un milieu αMEM-Chang indépendamment du nombre de cellules cultivées ;
- elles expriment les marqueurs HLA A, B, C ; CD10 ; CD29 ; CD44 ; CD73 ; CD90 ; CD105, SSEA4, OCT4, SOX2, NANOG, KLF4, cMyc, FGF4, FGF5, Nodal, ALP et n'expriment pas les marqueurs de classe II DR ; CD11b ; CD14 ; CD34 ; CD38 ; CD49d ; SSEA3 ;
- elles expriment des messagers pour CD117 ²(c-kit) ;
- elles expriment à la fois l'enzyme IDO1 et IDO2 ;
- elles expriment un niveau plus élevé de l'enzyme IDO en comparaison avec des lignées de cellules hématopoïétiques après des stimuli pro-inflammatoires.

3. Cellules souches pluripotentes selon la revendication 2, lesdites cellules souches pluripotentes étant en outre **caractérisées en ce que** :
- elles ne présentent aucun changement en termes de viabilité des cellules à la suite de procédures de culture *in vitro* ;
- elles peuvent être mises en culture en continu sur plus de 150 générations ;
- elles ne sont pas sujettes à des modifications phénotypiques après un cycle de congélation-décongélation ;
- elles présentent une stabilité chromosomique à la suite de procédures de culture *in vitro* ainsi que d'un cycle de congélation-décongélation ;
- elles ne présentent pas de tumorigénicité ;
- elles peuvent être stimulées par des molécules-signaux sphingosine-1-phosphate pour produire IDO et TGFβ1 ;
- à la suite d'une stimulation par INFγ, elles présentent une réduction en pourcentage des cellules de l'apoptose, une augmentation en pourcentage des cellules qui se dupliquent et une augmentation de l'expression de KLF4.

4. Composition pharmaceutique comprenant ou constituée par des cellules souches pluripotentes selon l'une des revendications 2 ou 3, conjointement avec un ou plusieurs excipients et/ou adjuvants pharmaceutiquement acceptables.

5. Composition pharmaceutique selon la revendication 4 sous la forme d'un implant.

6. Composition pharmaceutique selon l'une des revendications 4 ou 5, comprenant en outre des métabolites de tryptophane dérivés des cellules souches pluripotentes selon l'une des revendications 2 ou 3.

7. Cellules souches pluripotentes ou composition pharmaceutique selon les revendications 2 ou 3 et 4 à 6, respectivement, en vue d'une utilisation dans le domaine médical et vétérinaire.

8. Cellules souches pluripotentes ou composition pharmaceutique selon les revendications 2 ou 3 et 4 à 6 respectivement, en vue d'une utilisation dans le traitement ou la prévention de maladies auto-immunes.

9. Cellules souches pluripotentes ou composition pharmaceutique en vue d'une utilisation selon la revendication 8, dans lesquelles les maladies auto-immunes inflammatoires sont sélectionnées dans le groupe constitué par le diabète, la polyarthrite rhumatoïde, la sclérose en plaques.

10. Cellules souches pluripotentes ou composition pharmaceutique selon les revendications 2 ou 3 et 4 à 6, respectivement, en vue d'une utilisation dans la prévention du rejet d'une greffe d'organe.
